# EUROPÄISCHE PATENTSCHRIFT

(11) **EP 3 212 156 B1**
(45) Veröffentlichungstag und Bekanntmachung des Hinweises auf die Patenterteilung: **11.12.2019**
(21) Anmeldenummer: 15787229.2
(22) Anmeldetag: 30.10.2015
(51) Int. Cl.: A61K 8/365, A61Q 15/00, A61K 8/34

(54) **KOSMETISCHE ANTITRANSPIRANT-ZUSAMMENSETZUNG**
COSMETIC ANTIPERSPIRANT COMPOSITION
COMPOSITION ANTITRANSPIRANTE COSMÉTIQUE

(30) Priorität: 31.10.2014 DE 102014222270
(43) Veröffentlichungstag der Anmeldung: 06.09.2017
(73) Patentinhaber: Thomas Brunner Hygiene GmbH, 73278 Schlierbach (DE)
(72) Erfinder: BRUNNER, Thomas jr., 73278 Schlierbach (DE)
(74) Vertreter: Patentanwälte Ruff, Wilhelm, Beier, Dauster & Partner mbB
(86) Internationale Anmeldenummer: PCT/EP2015/075253
(87) Internationale Veröffentlichungsnummer: WO 2016/066801

(56) Entgegenhaltungen:
- WO-A1-01/52806
- WO-A1-95/30405
- DE-C1- 19 642 874
- US-A- 4 089 942
- US-A1- 2012 244 097
- DATABASE GNPD [Online] MINTEL; Februar 2010 (2010-02), Beiersdorf: "Deodorant Sheet", XP002751523, Database accession no. 1327273
- DATABASE GNPD [Online] MINTEL; August 2014 (2014-08), IP Manufacturing: "Antiperspirant Deodorant Roll On", XP002751524, Database accession no. 2594307

## Beschreibung

### Anwendungsgebiet und Stand der Technik

Die Erfindung betrifft eine kosmetische Antitranspirant-Zusammensetzung sowie ein kosmetisches Produkt.

Unter Antitranspirantien werden allgemein Substanzen verstanden, welche die Aktivität der Schweißdrüsen sowie die Menge des abgegebenen Schweißes reduzieren und somit indirekt den Körpergeruch vermindern.

Die Wirkungsweise von handelsüblichen Antitranspirant-Zusammensetzungen beruht auf der Anwesenheit von Aluminiumsalzen, wie beispielsweise Aluminiumchlorhydraten, als Antitranspirant-Wirkstoffe. Die Aluminiumsalze wirken dabei adstringierend und denaturierend. Durch die adstringierende Wirkung kommt es zu einer Verengung der Schweißkanäle. Aufgrund der denaturierenden Wirkung kommt es zu einer Ausfällung der Schweißproteine unter Ausbildung eines Proteinpfropfs am äußeren Ende der Schweißkanäle der Schweißdrüsen, wodurch insgesamt weniger Schweiß austreten kann.

Entsprechende Antitranspirant-Zusammensetzungen sind beispielsweise aus den Druckschriften DE 10 2011 086 019 A1, DE 10 2007 063 352 A1, EP 2 481 392 A2, WO 2001/047476 A2, WO 2003/041674 A1, WO 2005/112879 A1 sowie WO 2006/066704 A1 bekannt.

Aus der US 4,089,942 ist eine desodorierende Zusammensetzung bekannt. Die Zusammensetzung kann Ethanol sowie schwache organische Säuren, wie beispielsweise Weinsäure, enthalten.

Nachteilig bei den handelsüblichen Antitranspirant-Zusammensetzungen ist, dass die darin enthaltenen Aluminiumsalze im Verdacht stehen, dauerhafte Schäden im menschlichen Körper zu verursachen. So stehen Aluminiumsalze im Verdacht, Brustkrebs oder Alzheimer auszulösen.

Obgleich wissenschaftliche Belege, welche gesundheitliche Beeinträchtigungen durch aluminiumsalzhaltige Kosmetika zweifelsfrei stützen, bislang nicht zur Verfügung stehen, besteht ein Bedarf nach Antitranspirant-Zusammensetzungen, welche keinen medizinischen Bedenken begegnen.

### Aufgabe und Lösung

Der vorliegenden Erfindung lag daher die Aufgabe zugrunde, eine kosmetische Antitranspirant-Zusammensetzung sowie ein kosmetisches Produkt bereitzustellen, welche den oben genannten Bedarf adressieren und aus dem Stand der Technik bekannte Nachteile umgehen.

Diese Aufgabe wird gelöst durch eine kosmetische Antitranspirant-Zusammensetzung mit den Merkmalen des Anspruchs 1 sowie durch ein kosmetisches Produkt gemäß Anspruch 10. Bevorzugte Ausführungsformen sind in den abhängigen Ansprüchen 2 bis 9 definiert. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung eine kosmetische Antitranspirant-Zusammensetzung, d.h. eine transpirations- bzw. schweißhemmende Zusammensetzung, welche wenigstens einen Antitranspirant-Wirkstoff enthält, welcher ausgewählt ist aus der Gruppe bestehend aus Weinsäure, Weinsäuresalz und Mischungen davon.

Der vorliegenden Erfindung liegt die überraschende Erkenntnis zugrunde, dass die im Folgenden noch näher erläuterten Hydroxycarbonsäuren bzw. Hydroxycarbonsäuresalze geeignet sind, in handelsüblichen Antitranspirant-Zusammensetzungen enthaltene Aluminiumsalze zu substituieren.

Die kosmetische Antitranspirant-Zusammensetzung reduziert in vorteilhafter Weise insbesondere die Aktivität von apokrinen Schweißdrüsen, welche insbesondere im Bereich der Axilla (pyramidenförmiger anatomischer Raum zwischen der lateralen Brustwand und dem medialen Oberarm), der Brustwarze, in der Leistengegend, der perianalen Region (Glandulae cirumanales) und der Schamregion (Frau: Mons pupis, große Schamlippen; Mann: Hodensack) lokalisiert sind.

Der Begriff "Hydroxycarbonsäure" kann im Sinne der vorliegenden Erfindung sowohl einen Hydroxycarbonsäuretyp als auch mehrere verschiedene Hydroxycarbonsäuretypen bedeuten. Entsprechend kann der Begriff "Hydroxycarbonsäuresalz" im Sinne der vorliegenden Erfindung einen Hydroxycarbonsäuresalztyp oder mehrere verschiedene Hydroxycarbonsäuresalztypen bedeuten.

Die im Folgenden in Bezug auf die Hydroxycarbonsäure gemachten Ausführungen gelten sinngemäß auch für das Hydroxycarbonsäuresalz.

In einer bevorzugten Ausführungsform ist die kosmetische Antitranspirant-Zusammensetzung frei von Zirkoniumsalzen. Mit anderen Worten ist es erfindungsgemäß bevorzugt, dass die kosmetische Antitranspirant-Zusammensetzung zirkoniumsalzfrei ist.

Gemäß einer weitergehenden Ausführungsform ist die kosmetische Antitranspirant-Zusammensetzung aluminium-, zirkonium- und gegebenenfalls zinksalzfrei.

Die Antitranspirant-Zusammensetzung ist aluminiumsalzfrei, d.h. frei von Aluminiumsalzen. Mit anderen Worten handelt es sich bei der erfindungsgemäßen Antitranspirant-Zusammensetzung um eine aluminiumsalzfreie, kosmetische Antitranspirant-Zusammensetzung. Der Ausdruck "Aluminiumsalz" definiert im Sinne der vorliegenden Erfindung allgemein aluminiumhaltige Salze und umfasst mithin sowohl anorganische als auch organische Salze des Aluminiums, wie beispielsweise Aluminiumchlorhydrate und Aluminiumhydroxylactate, sowie auch Mischsalze des Aluminiums mit anderen Elementen, wie beispielsweise Zirkonium.

In einer weitergehenden Ausführungsform ist die Antitranspirant-Zusammensetzung frei von Aluminiumverbindungen. Mit anderen Worten kann es sich bei der erfindungsgemäßen Antitranspirant-Zusammensetzung weiterhin bevorzugt um eine aluminiumfreie, kosmetische Antitranspirant-Zusammensetzung handeln. Der Ausdruck "Aluminiumverbindungen" definiert im Sinne der vorliegenden Erfindung allgemein Verbindungen des chemischen Elements Aluminium. Unter dem Ausdruck "Aluminiumverbindungen" werden im Sinne der vorliegenden Erfindung somit nicht nur Aluminiumsalze, sondern insbesondere auch aluminiumhaltige Komplexverbindungen, wie beispielsweise Aluminium-Zirkonium-Tetrachlorohydrex-Glycin-Komplex, verstanden.

In einer weiteren Ausführungsform enthält die Antitranspirant-Zusammensetzung neben dem erfindungsgemäß vorgesehenen wenigstens einen Antitranspirant-Wirkstoff keinen zusätzlichen Antitranspirant-Wirkstoff.

Der wenigstens eine Antitranspirant-Wirkstoff ist ausgewählt ist aus der Gruppe bestehend aus Weinsäure, Salze davon und Mischungen davon. Mit anderen Worten handelt es sich bei dem wenigstens einen Antitranspirant-Wirkstoff um Weinsäure, Salze davon und/oder Mischungen davon.

Erfindungsgemäß kann es weiterhin bevorzugt sein, wenn die kosmetische Antitranspirant-Zusammensetzung als Antitranspirant-Wirkstoff/Antitranspirant-Wirkstoffe nur Weinsäure, Zitronensäure, Äpfelsäure, Gallussäure, Salze davon und/oder Mischungen davon enthält.

Erfindungsgemäß handelt es sich bei dem wenigstens einen Antitranspirant-Wirkstoff um Weinsäure oder ein Weinsäuresalz. Die erfindungsgemäß erläuterten Vorteile kommen im Falle von Weinsäure bzw. einem Weinsäuresalz besonders stark zur Geltung.

Der wenigstens eine Antitranspirant-Wirkstoff weist einen Anteil von 2.5 Gew.-% bis 40 Gew.-%, insbesondere 3 Gew.-% bis 35 Gew.-%, bevorzugt 5 Gew.-% bis 30 Gew.-%, besonders bevorzugt 8 Gew.-% bis 25 Gew.-%, höchst bevorzugt 10 Gew.-% bis 20 Gew.-%, auf, bezogen auf das Gesamtgewicht der Zusammensetzung. Der Erfindung liegt weiterhin die überraschende Erkenntnis zugrunde, dass die denaturierenden Eigenschaften des erfindungsgemäß vorgesehenen wenigstens einen Antitranspirant-Wirkstoffs bei den in diesem Absatz genannten Konzentrationen besonders stark zur Geltung kommen. Eine weitere vorteilhafte Wirkung der in diesem Absatz beschriebenen Antitranspirant-Wirkstoffanteile besteht darin, dass ein Zusatz von Konservierungsstoffen entbehrlich ist.

Die Weinsäure kann in einer Form vorliegen, welche ausgewählt ist aus der Gruppe bestehend aus D-(-)-Weinsäure, L-(+)-Weinsäure, Meso-Weinsäure, Salze davon und Mischungen davon. Insbesondere kann die Weinsäure in Form einer racemischen Mischung aus D-(-)-Weinsäure (bzw. einem Salz davon) und L-(+)-Weinsäure (bzw. einem Salz davon) vorliegen.

Die kosmetische Antitranspirant-Zusammensetzung enthält ferner wenigstens ein Trägerfluid. Unter dem Ausdruck "Trägerfluid" soll im Sinne der vorliegenden Erfindung ein flüssiges Trägermaterial, insbesondere ein Dispersions-, Lösungs- oder Suspensionsmittel, verstanden werden. Das Trägerfluid ist Ethanol und kann zusätzlich aus der Gruppe bestehend aus Isopropanol, 2-Methyl-propanol-2, Wasser und Mischungen davon ausgewählt sein.

Die Verwendung von Ethanol als Trägerfluid ist besonders vorteilhaft, da Ethanol insbesondere in höheren Konzentrationen stark denaturierend auf Proteinbausteine wirkt. Hierdurch lassen sich die transpirationshemmenden Eigenschaften der erfindungsgemäßen Zusammensetzung zusätzlich verstärken. Außerdem wirkt Ethanol vor allem in höheren Konzentrationen antibakteriell, wodurch auf der Haut befindliche Bakterien, welche den Schweiß zersetzen und deren Ausscheidungen ursächlich für Körper- bzw. Schweißgeruch sind, abgetötet werden können. Auf diese Weise können die körper- bzw. schweißgeruchsbekämpfenden Eigenschaften der erfindungsgemäßen Zusammensetzung zusätzlich verstärkt werden. Ein weiterer Vorteil von Ethanol besteht darin, dass ein Zusatz von Konservierungsstoffen nicht erforderlich ist.

Ein geeignetes ethanolisches Trägerfluid stellt beispielsweise das unter dem Handelsnamen Cosmetol (96%ig) kommerziell erhältliche Produkt dar. Dieses Produkt besitzt einen Ethanolanteil von 96 Gew.-%, einen Isopropanolanteil von 2 Gew.-% sowie einen Anteil an 2-Methyl-propanol-2 von 2 Gew.-%, jeweils bezogen auf das Gesamtgewicht des Produkts.

Ein weiterer Vorteil in der Verwendung von Ethanol als Trägerfluid besteht darin, dass es zusammen mit dem erfindungsgemäß vorgesehenen wenigstens einen Antitranspirant-Wirkstoff einen synergistischen Wirkkomplex bildet, dessen transpirationshemmende Wirkung derjenigen einer wasserbasierten, aluminiumsalzhaltigen Kosmetik-Formulierung entspricht.

Die erfindungsgemäße Zusammensetzung weist einen Ethanolanteil von 10 Gew.-% bis 50 Gew.-%, insbesondere 15 Gew.-% bis 40 Gew.-%, bevorzugt 20 Gew.-% bis 30 Gew.-%, auf, bezogen auf das Gesamtgewicht der Zusammensetzung. Die synergistische Wirkung von Ethanol im Zusammenspiel mit dem erfindungsgemäß vorgesehenen, wenigstens einen Antitranspirant-Wirkstoff kommt bei in diesem Absatz genannten Konzentrationen besonders gut zur Geltung.

Die kosmetische Antitranspirant-Zusammensetzung weist in einer weiteren Ausführungsform einen Wasseranteil von 10 Gew.-% bis 75 Gew.-%, bevorzugt 35 Gew.-% bis 50 Gew.-%, auf, bezogen auf das Gesamtgewicht der Zusammensetzung.

In einer weiteren Ausführungsform enthält die kosmetische Antitranspirant-Zusammensetzung ferner Saccharomyces Ferment. Hierbei handelt es sich um ein Endprodukt der Fermentation durch Saccharomyces, d.h. um ein Zuckerhefen-Ferment. Ein derartiges Ferment ist beispielsweise unter dem Handelsnamen Deosent, DeoPlex Clear H5630 kommerziell erhältlich. Saccharomyces Ferment besitzt mit besonderem Vorteil eine geruchsüberdeckende bzw. geruchsmindernde Wirkung. Diese Wirkung beruht auf einer enzymatischen Umsetzung bestimmter Riechstoffgruppen, wodurch entsprechende Geruchs- bzw. Duftstoffe zu größeren, schwereren Verbindungen umgesetzt werden, welche aufgrund ihres höheren Gewichts nicht mehr so leicht verdampfen können und daher nicht mehr geruchlich wahrgenommen werden.

Bevorzugt weist das Saccharomyces Ferment einen Anteil von 0.5 Gew.-% bis 5 Gew.-%, insbesondere 0.75 Gew.-% bis 4 Gew.-%, bevorzugt 1 Gew.-% bis 3 Gew.-%, auf, bezogen auf das Gesamtgewicht der Zusammensetzung.

In einer weiteren Ausführungsform enthält die kosmetische Antitranspirant-Zusammensetzung ferner wenigstens einen pH-Wert-Regulator. Bevorzugt handelt es sich bei dem pH-Wert-Regulator um eine Verbindung, welche gegenüber den erfindungsgemäß vorgeschlagenen Antitranspirant-Wirkstoffen, insbesondere gegenüber Weinsäure, inert ist. Beispielsweise kann es sich bei dem pH-Wert-Regulator um Triethanolamin handeln.

Bevorzugt weist der wenigstens eine pH-Wert-Regulator einen Anteil von 0 Gew.-% bis 2.5 Gew.-%, insbesondere 0.01 Gew.-% bis 2.5 Gew.-%, bevorzugt 2 Gew.-% bis 2.5 Gew.-% auf, bezogen auf das Gesamtgewicht der Zusammensetzung.

In einer weiteren Ausführungsform enthält die kosmetische Antitranspirant-Zusammensetzung ferner wenigstens einen Komplexbildner. Bevorzugt handelt es sich bei dem wenigstens einen Komplexbildner um Polyphosphate. Beispielsweise kann der wenigstens eine Komplexbildner ausgewählt sein aus der Gruppe bestehend aus Natriumpolyphosphat, Natrium-Hexametaphosphat, Kaliumpolyphosphat, Calciumpolyphosphat, Natriumcalciumpolyphosphat und Mischungen davon.

Bevorzugt weist der wenigstens eine Komplexbildner einen Anteil von 0 Gew.-% bis 2.5 Gew.-%, insbesondere 0.01 Gew.-% bis 2.5 Gew.-%, bevorzugt 0.02 Gew.-% bis 0.8 Gew.-%, auf, bezogen auf das Gesamtgewicht der Zusammensetzung.

In einer weiteren Ausführungsform enthält die kosmetische Antitranspirant-Zusammensetzung ferner wenigstens einen Viskositätsregulator bzw. Filmbildner. Der wenigstens eine Viskositätsregulator bzw. Filmbildner kann aus der Gruppe bestehend aus Methylhydroxyethylcellulose, Carboxymethylcellulose, Methylcellulose, Ethylcellulose, Hydroxyethylcellulose, Hydroxyethylmethylcellulose, Hydroxypropylmethylcellulose, homopolymere Polyacrylsäure (sogenannte Carbomere), heteropolymere Polyacrylsäure bzw. Acrylat-Einheiten enthaltende Copolymere, Guaran, Guar Gummi, Hydroxypropyl Guar (Guar Gummi, 2-Hydroxypropylether, depolymerisiert) wie Esaflor® HDR, Carboxymethyl Guar und Mischungen davon ausgewählt sein.

Bevorzugt weist der wenigstens eine Viskositätsregulator bzw. Filmbildner einen Anteil von 0 Gew.-% bis 4 Gew.-%, bevorzugt 0.01 Gew.-% bis 2 Gew.-%, insbesondere 0.01 Gew.-% bis 0,5 Gew.-%, auf, bezogen auf das Gesamtgewicht der Zusammensetzung.

Um eine verbesserte Durchdringung der Hautbarriere, d.h. eine verbesserte dermale Aufnahme, zu erzielen, enthält die kosmetische Antitranspirant-Zusammensetzung in einer weiteren Ausführungsform ferner wenigstens ein Tensid, insbesondere wenigstens ein emulgierendes Tensid.

Das wenigstens eine Tensid kann ausgewählt sein aus der Gruppe bestehend aus Polyoxyethylen(20)-sorbitan-monolaurat, Kokosglucoside wie Plantacare 818, Cocamidopropylbetain, Coco-Betaine wie Dehyton PK 45 und/oder Tego Betain F50 und Mischungen davon. Polyoxyethylen(20)-sorbitan-monolaurat ist beispielsweise unter dem Handelsnahmen Polysorbat 20 kommerziell erhältlich.

Bevorzugt weist das wenigstens eine Tensid einen Anteil von 0 Gew.-% bis 2 Gew.-%, insbesondere 0.01 Gew.-% bis 2 Gew.-%, bevorzugt 0.2 Gew.-% bis 1 Gew.-%, auf, bezogen auf das Gesamtgewicht der Zusammensetzung.

In einer unter Hautpflegegesichtspunkten vorteilhaften Ausführungsform enthält die kosmetische Antitranspirant-Zusammensetzung ferner wenigstens einen Hautpflegestoff, insbesondere wenigstens ein Feuchthalte- und/oder Hautbefeuchtungsmittel.

Der wenigstens eine Hauptpflegestoff kann ausgewählt sein aus der Gruppe bestehend aus Aminosäuren, Milchsäure und deren Salze, Lactitol, Harnstoff, Harnstoffderivate, Harnsäure, Glucosamin, Kreatinin, Spaltprodukte des Kollagens, Chitosan, Chitosansalze/-derivate, Polyole und Polyolderivate, wie beispielsweise Glycerin, Diglycerin, Allantoin, Dexpanthenol, Triglycerin, Glycerinester wie Glycerycaprylat, Ethylenglycol, Propylenglycol, Butylenglycol, Erythrit, 1,2,6-Hexantriol, Polyethylenglycole, wie beispielsweise PEG-4, PEG-6, PEG-7, PEG-8, PEG-9, PEG-10, PEG-12, PEG-14, PEG-16, PEG-18, PEG-20, Zucker und Zuckerderivate, wie beispielsweise Fructose, Glucose, Maltose, Maltit, Mannit, Inosit, Sorbit, Sorbitylsilandiol, Sucrose, Trehalose, Xylose, Xylit, Glucuronsäure und deren Salze, sowie Mischungen davon.

Bevorzugt ist der wenigstens eine Hautpflegestoff ausgewählt aus der Gruppe bestehend aus Harnstoff, Propan-1,2-diol, Glycerin, Diglycerin, Allantoin, Dexpanthenol und Mischungen davon.

Der wenigstens eine Hautpflegestoff weist vorzugsweise einen Anteil von 0 Gew.-% bis 20 Gew.-%, insbesondere 0.01 Gew.-% bis 20 Gew.-%, bevorzugt 6 Gew.-% bis 17 Gew.-%, auf, bezogen auf das Gesamtgewicht der Zusammensetzung.

Beispielsweise kann die kosmetische Antitranspirant-Zusammensetzung einen Harnstoffanteil von 0 Gew.-% bis 5 Gew.-%, insbesondere 0.01 Gew.-% bis 5 Gew.-%, bevorzugt 1 Gew.% bis 3 Gew.-%, aufweisen, bezogen auf das Gesamtgewicht der Zusammensetzung.

Weiterhin kann die kosmetische Antitranspirant-Zusammensetzung einen Anteil an Propan-1,2-diol von 0 Gew.-% bis 10 Gew.-%, insbesondere 0.01 Gew.-% bis 10 Gew.-%, bevorzugt 2 Gew.-% bis 6 Gew.-%, aufweisen, bezogen auf das Gesamtgewicht der Zusammensetzung.

Weiterhin kann die kosmetische Antitranspirant-Zusammensetzung einen Glycerinanteil von 0 Gew.-% bis 10 Gew.-%, insbesondere 0.01 Gew.-% bis 10 Gew.-%, bevorzugt 2 Gew.-% bis 6 Gew.-%, aufweisen, bezogen auf das Gesamtgewicht der Zusammensetzung.

Weiterhin kann die kosmetische Antitranspirant-Zusammensetzung einen Allantoinanteil von 0 Gew.-% bis 0.5 Gew.-%, insbesondere 0.01 Gew.-% bis 0.5 Gew.-%, bevorzugt 0.3 Gew.-% bis 0.5 Gew.-%, aufweisen, bezogen auf das Gesamtgewicht der Zusammensetzung.

Weiterhin kann die kosmetische Antitranspirant-Zusammensetzung einen Dexpanthenolanteil von 0 Gew.-% bis 5 Gew.-%, insbesondere 0.01 Gew.-% bis 5 Gew.-%, bevorzugt 0.5 Gew.-% bis 1 Gew.-%, aufweisen, bezogen auf das Gesamtgewicht der Zusammensetzung.

In einer unter duftneutralisierenden Gesichtspunkten vorteilhaften Ausführungsform enthält die kosmetische Antitranspirant-Zusammensetzung ferner wenigstens einen desodorierenden Wirkstoff. Der wenigstens eine desodorierende Wirkstoff kann ausgewählt sein aus der Gruppe bestehend aus Duft- bzw. Geruchsstoffe, Parfüm (flüssiges Gemisch aus Alkohol, insbesondere Ethanol, und Geruchs- bzw. Duftstoffen) Geruchsabsorber oder -löscher, antimikrobielle Wirkstoffe, Enzyminhibitoren und Mischungen davon.

Beispielsweise kann die kosmetische Antitranspirant-Zusammensetzung ferner wenigstens einen Duft- bzw. Geruchsstoff enthalten. Hinsichtlich des wenigstens einen Duft- bzw. Geruchsstoffes bestehen grundsätzlich keinerlei Beschränkungen, solange es sich hierbei um einen Duft- bzw. Geruchsstoff handelt, welcher eine leicht saure Umgebung toleriert. Beispielsweise kann es sich bei dem wenigstens einen Duft- bzw. Geruchsstoff um Menthol handeln.

Bevorzugt weist die kosmetische Antitranspirant-Zusammensetzung einen Duft- bzw. Geruchsstoffanteil von 0 Gew.-% bis 0.2 Gew.-%, insbesondere 0.01 Gew.-% bis 0.2 Gew.-%, bevorzugt 0.05 Gew.-% bis 0.1 Gew.-%, auf, bezogen auf das Gesamtgewicht der Zusammensetzung.

Geeignete Geruchsabsorber bzw. -löscher stellen Silikate, vor allem Schichtsilikate, wie beispielsweise Montmorillonit, Kaolinit, Beidellit, Saponit, Bentonit, Zeolithe, Cyclodextrine und Mischungen davon, dar.

Geeignete antimikrobielle Wirkstoffe können ausgewählt sein aus der Gruppe bestehend aus Triclosan, Chlorhexidin, Chlorhexidingluconat, Bromchlorophen, Dichlorophen, Chlorothymol, Chloroxylenol, Hexachlorophen, Ammoniumphenolsulfonat, Benzalkoniumhalogenide, Benzalkoniumcetylphosphat, Benzalkoninumsaccharinat, Benzethoniumchlorid, Cetylpyridiniumchlorid, Laurylpyridiniumchlorid, Laurylisoquinoliniumbromid, Methylbenzethoniumchlorid, Terpenalkohole, wie beispielsweise Farnesol, Chlorophyllin-Kupfer-Komplexe, Carbonsäureester von Mono-, Di- und/oder Triglycerin, wie beispielsweise Glycerinmonolaurat und/oder Diglycerinmonocaprinat, Fettsäuren, Pflanzenextrakte, wie beispielsweise grüner Tee und/oder Bestandteil des Lindenblütenöls, und Mischungen davon.

In einer weiteren Ausführungsform enthält die kosmetische Antitranspirant-Zusammensetzung ferner wenigstens einen Konservierungsstoff. Der wenigstens eine Konservierungsstoff kann ausgewählt sein aus der Gruppe bestehend aus Benzoesäure und deren Derivate, Propionsäure und deren Derivate, Salicylsäure und deren Derivate, Alkohole und deren Derivate, Guajacol und dessen Derivate, Hydantoin und dessen Derivate, Ferulasäure und deren Derivate, Sorbinsäure und deren Derivate und Mischungen davon.

Erfindungsgemäß kann es vorgesehen sein, dass die kosmetische Antitranspirant-Zusammensetzung einen Konservierungsstoffanteil von 0.01 Gew.-% bis 1 Gew.-%, bevorzugt 0.1 Gew.-% bis 0.5 Gew-%, aufweist, bezogen auf das Gesamtgewicht der Zusammensetzung.

In einer alternativen Ausführungsform ist die kosmetische Antitranspirant-Zusammensetzung frei von einem Konservierungsstoff. Wie bereits erwähnt, kann aufgrund der Verwendung wenigstens eines der vorgeschlagenen Antitranspirant-Wirkstoffe und/oder der Verwendung von Ethanol als Trägerfluid ein Zusatz von Konservierungsstoffen entbehrlich sein.

In einer weiteren Ausführungsform enthält die kosmetische Antitranspirant-Zusammensetzung ferner wenigstens ein Antioxidans. Das wenigstens eine Antioxidans kann ausgewählt sein aus der Gruppe bestehend aus Aminosäuren, wie beispielsweise Glycin, Histidin, Tyrosin, Tryptophan, Aminosäurederivate, Imidazol, Imidazolderivate, wie beispielsweise Urocaninsäure, Carotinoide, Carotine, wie beispielsweise α-Carotin, β-Carotin und/oder Lycopin, Carotinoid-Derivate, Carotin-Derivate, Liponsäure und deren Derivate, wie beispielsweise Dihydroliponsäure, Thioverbindungen, Huminsäuren, Gallensäure, Gallenextrakte, Flavonoide, Katechine, Bilirubin, Biliverdin, ungesättigte Fettsäuren und deren Derivate, wie beispielsweise γ-Linolensäure, Linolsäure, Arachidonsäure, Ölsäure, Folsäure und deren Derivate, Hydrochinon und dessen Derivate, Ubichinon und Ubichinol sowie deren Derivate, Vitamin C und dessen Derivate, Tocopherole und deren Derivate und Mischungen davon.

Erfindungsgemäß kann es vorgesehen sein, dass die kosmetische Antitranspirant-Zusammensetzung einen Antioxidansanteil von 0.01 Gew.-% bis 1 Gew.-%, bevorzugt 0.1 Gew.-% bis 0.5 Gew.-%, aufweist, bezogen auf das Gesamtgewicht der Zusammensetzung.

In einer weiteren Ausführungsform enthält die kosmetische Antitranspirant-Zusammensetzung ferner wenigstens eine Zinkverbindung, insbesondere wenigstens ein Zinksalz, wie beispielsweise Zinkoxid.

In einer weiteren Ausführungsform enthält die kosmetische Antitranspirant-Zusammensetzung ferner wenigstens eine antiadhäsive Verbindung. Bei der wenigstens einen antiadhäsiven Verbindung kann es sich insbesondere um silikonbasierte Verbindungen, wie beispielsweise Cyclopentasiloxane, handeln.

In einer bevorzugten Ausführungsform enthält die Zusammensetzung auf 100 Gew-% folgende Inhaltsstoffe:
- 5 Gew.-% bis 30 Gew.-%, bevorzugt 10 Gew.-% bis 20 Gew.-%, des wenigstens einen Antitranspirant-Wirkstoffes,
- 10 Gew.-% bis 50 Gew.-%, bevorzugt 20 Gew.-% bis 30 Gew.-%, Ethanol,
- 0.5 Gew.-% bis 5 Gew.-%, bevorzugt 1 Gew.-% bis 3 Gew.-%, Saccharomyces Ferment,
- 0 Gew.-% bis 2.5 Gew.-%, bevorzugt 2 Gew.-% bis 2.5 Gew.-%, wenigstens eines pH-Regulators,
- 0 Gew.-% bis 2.5 Gew.-%, bevorzugt 0.2 Gew.-% bis 0.8 Gew.-%, wenigstens eines Komplexbildners,
- 0 Gew.-% bis 4 Gew.-%, bevorzugt 0.01 Gew.-% bis 2 Gew.-%, wenigstens eines Viskositätsregulators bzw. Filmbildners,
- 0 Gew.-% bis 2 Gew.-%, bevorzugt 0.2 Gew.-% bis 1 Gew.-%, wenigstens eines Tensids,
- 0 Gew.-% bis 30.5 Gew.-%, bevorzugt 5.5 Gew.-% bis 16.5 Gew.-%, wenigstens eines Hautpflegestoffes,
- 0 Gew.-% bis 0.2 Gew.-%, bevorzugt 0.05 Gew.-% bis 0.1 Gew.-%, wenigstens eines Duftstoffes und
- 10 Gew.-% bis 75 Gew.-%, bevorzugt 35 Gew.-% bis 50 Gew.-%, Wasser.

In einer besonders bevorzugten Ausführungsform enthält die Zusammensetzung auf 100 Gew.-% folgende Inhaltsstoffe:
- 5 Gew.-% bis 30 Gew.-%, bevorzugt 10 Gew.-% bis 20 Gew.-%, Weinsäure oder Weinsäuresalz,
- 10 Gew.-% bis 50 Gew.-%, bevorzugt 20 Gew.-% bis 30 Gew.-%, Ethanol,
- 0.5 Gew.-% bis 5 Gew.-%, bevorzugt 1 Gew.-% bis 3 Gew.-%, Saccharomyces Ferment,
- 0 Gew.-% bis 2.5 Gew.-%, bevorzugt 2 Gew.-% bis 2.5 Gew.-%, Triethanolamin,
- 0 Gew.-% bis 2.5 Gew.-%, bevorzugt 0.2 Gew.-% bis 0.8 Gew.-%, Natriumpolyphosphat,
- 0 Gew.-% bis 4 Gew.-%, bevorzugt 0.01 Gew.-% bis 2 Gew.-%, Methylhydroxyethylcellulose oder Hydroxypropyl Guar,
- 0 Gew.-% bis 2 Gew.-%, bevorzugt 0.2 Gew.-% bis 1 Gew.-%, Polyoxyethylen(20)-sorbitan-monolaurat,
- 0 Gew.-% bis 5 Gew.-%, bevorzugt 1 Gew.-% bis 3 Gew.-%, Harnstoff,
- 0 Gew.-% bis 10 Gew.-%, bevorzugt 2 Gew.-% bis 6 Gew.-%, Propan-1,2-diol,
- 0 Gew.-% bis 10 Gew.-%, bevorzugt 2 Gew.-% bis 6 Gew.-%, Glycerin,
- 0 Gew.-% bis 0.5 Gew.-%, bevorzugt 0.3 Gew.-% bis 0.5 Gew.-%, Allantoin,
- 0 Gew.-% bis 5 Gew.-%, bevorzugt 0.5 Gew.-% bis 1 Gew.-%, Dexpanthenol,
- 0 Gew.-% bis 0.2 Gew.-%, bevorzugt 0.05 Gew.-% bis 0.1 Gew.-%, Menthol oder Parfüm und
- 10 Gew.-% bis 75 Gew.-%, bevorzugt 35 Gew.-% bis 50 Gew.-%, Wasser.

In einer weiteren Ausführungsform besteht die kosmetische Antitranspirant-Zusammensetzung aus wenigstens einem der erfindungsgemäß vorgesehenen Antitranspirant-Wirkstoffe sowie aus wenigstens einem weiteren Inhaltsstoff, welcher vorzugsweise ausgewählt ist aus der Gruppe bestehend aus Trägerfluide, pH-Wert-Regulatoren, Komplexbildner, Viskositätsregulatoren bzw. Filmbildner, Tenside, Hautpflegestoffe, Konservierungsstoffe, Antioxidantien, Zinkverbindungen, antiadhäsive Verbindungen und Mischungen davon. Bezüglich des wenigstens einen erfindungsgemäß vorgesehenen Antitranspirant-Wirkstoffes sowie der Inhaltsstoffe wird vollständig auf die bisherige Beschreibung Bezug genommen.

Erfindungsgemäß kann es beispielsweise vorgesehen sein, dass die kosmetische Antitranspirant-Zusammensetzung aus wenigstens einem der erfindungsgemäß vorgesehenen Antitranspirant-Wirkstoffe, wenigstens einem Trägerfluid, wenigstens einem pH-Wert-Regulator, wenigstens einem Komplexbildner, wenigstens einem Viskositätsregulator bzw. Filmbildner, wenigstens einem Tensid, wenigstens einem Hautpflegestoff, wenigstens einem Duft- bzw. Geruchsstoff und gegebenenfalls wenigstens einer antiadhäsiven Verbindung und/oder wenigstens einer Zinkverbindung besteht. Bezüglich des wenigstens einen erfindungsgemäß vorgesehenen Antitranspirant-Wirkstoffes sowie der Inhaltsstoffe wird ebenfalls vollständig auf die bisherige Beschreibung Bezug genommen.

In einer weiteren Ausführungsform ist die kosmetische Antitranspirant-Zusammensetzung als Lösung, Suspension, Gel, insbesondere Hydrogel, Creme, Paste, Salbe, Roll-on-Zusammensetzung, Stift, Spray-Sud, Pumpspray oder zusammen mit einem Treibmittel als Aerosolspray konfektioniert.

Mit anderen Worten ist es erfindungsgemäß bevorzugt, wenn die Antitranspirant-Zusammensetzung in Form einer Lösung, einer Suspension, eines Gels, insbesondere Hydrogels, einer Creme, einer Paste, einer Salbe, einer Roll-on-Zusammensetzung, eines Stifts, eines Spray-Suds, eines Pumpsprays oder zusammen mit einem Treibmittel in Form eines Aerosolsprays vorliegt.

Gemäß einem zweiten Aspekt betrifft die Erfindung ein kosmetisches Produkt, welches eine Antitranspirant-Zusammensetzung gemäß erstem Erfindungsaspekt enthält oder aus einer solchen Zusammensetzung besteht.

Bei dem kosmetischen Produkt handelt es sich vorzugsweise um ein kosmetisches Gel wie kosmetisches Hydrogel, eine kosmetische Creme, eine kosmetische Paste, eine kosmetische Salbe, ein kosmetisches Roll-on-Produkt, einen kosmetischen Stift, ein kosmetisches Spray-Sud, ein kosmetisches Pumpspray oder ein kosmetisches Aerosolspray. Im Falle einer Ausgestaltung als kosmetisches Aerosolspray kann das kosmetische Produkt neben einer Antitranspirant-Zusammensetzung gemäß erstem Erfindungsaspekt ein geeignetes Treibmittel enthalten.

Bezüglich weiterer Merkmale und Vorteile des kosmetischen Produkts, insbesondere der Antitranspirant-Zusammensetzung, wird vollständig auf die im Rahmen des ersten Erfindungsaspektes gemachten Ausführungen Bezug genommen.

Bezüglich weiterer Merkmale und Vorteile der Hydroxycarbonsäure, dem Salz der Hydroxycarbonsäure sowie der kosmetischen Antitranspirant-Zusammensetzung wird vollständig auf die im Rahmen der bisherigen Erfindungsaspekte gemachten Ausführungen Bezug genommen.

Weitere Merkmale und Vorteile der Erfindung ergeben sich aus der nachfolgenden Beschreibung bevorzugter Ausführungsformen in Form von Beispielen sowie der Unteransprüche. Die bevorzugten Ausführungsformen dienen lediglich dem besseren Verständnis der Erfindung, ohne diese hierauf zu beschränken.

### Beispielteil

### 1. Herstellung von Antitranspirant-Zusammensetzungen

### 1.1 Herstellung einer transpirationshemmenden Zusammensetzung zur Verwendung als Pumpspray mit hoher Wirkdauer

In einen Rührbehälter wurden zunächst 225.625 g temperiertes Wasser (50°C), 0.500 g Natrium-Hexametaphosphat, 55.000 g Weinsäure und 12.500 g Triethanolamin (99%ig) hinzugegeben. Danach ließ man den Rührbehälter während eines Zeitraumes von 3 min. rühren.

Anschließend wurden 15.000 g Harnstoff, 2.500 g Allantoin, 30.000 g Propylenglycol (1.2 USP), 20.000 g Glycerin (99%ig) sowie 3.000 g Sorbital T 20 P hinzugegeben. Man ließ den Rührbehälter für weitere 6 min. rühren. Danach wurden 0.375 g Menthol (kristallin), 125.000 g Cosmetol (96%ig), 7.500 g Deosent/DeoPlex Clear und 3.000 g Dexpanthenol hinzugegeben. Man ließ den Rührbehälter abermals während eines Zeitraumes von 6 min. rühren.

Es wurde eine farblose, klare, nach Alkohol riechende sowie niedrigviskose Zusammensetzung erhalten, welche die obengenannten Inhaltsstoffe in den folgenden Anteilen aufwies:
45.12 Gew.-% Wasser
0.10 Gew.-% Natrium-Hexametaphosphat
11.00 Gew.-% Weinsäure
2.50 Gew.-% Triethanolamin (99%ig)
3.00 Gew.-% Harnstoff
0.50 Gew.-% Allantoin
6.00 Gew.-% Propylenglycol (1,2 USP)
4.00 Gew.-% Glycerin (99%ig)
0.60 Gew.-% Sorbital (T 20 P)
0.08 Gew.-% Menthol (kristallin)
25 Gew.-% Cosmetol (96%ig)
1.50 Gew.-% Deosent/DeoPlex Clear
0.60 Gew.-% Dexpanthenol.

### 1.2 Herstellung einer weiteren transpirationshemmenden Zusammensetzung zur Verwendung als Pumpspray mit 24 stündiger Wirkdauer

In einen Rührbehälter wurden zunächst 253.625 g temperiertes Wasser (50°C), 0.500 g Natrium-Hexametaphosphat, 27.500 g Weinsäure sowie 12.500 g Triethanolamin (99%ig) hinzugegeben. Danach ließ man den Rührbehälter während eines Zeitraumes von 3 min. rühren.

Anschließend wurden 15.000 g Harnstoff, 2.000 g Allantoin, 30.000 g Propylenglycol (1,2 USP), 20.000 g Glycerin (99%ig) sowie 3.000 g Sorbital T 20 P hinzugegeben. Danach ließ man den Rührbehälter während eines Zeitraumes von 6 min. rühren.

Anschließend wurden 0.375 g Menthol (kristallin), 125.000 g Cosmetol (96%ig), 7.5000 g Deosent/DeoPlex Clear sowie 3.000 g Dexpanthenol hinzugegeben. Man ließ den Rührbehälter abermals während eines Zeitraumes von 6 min. rühren.

Man erhielt eine farblose, klar aussehende, nach Alkohol riechende und mittelviskose Zusammensetzung, welche die obengenannten Inhaltsstoffe in den folgenden Anteilen aufwies:
50.72 Gew.-% Wasser
0.10 Gew.-% Natrium-Hexametaphosphat
5.50 Gew.-% Weinsäure
2.50 Gew.-% Triethanolamin (99%ig)
3.00 Gew.-% Harnstoff
0.40 Gew.-% Allantoin
6.00 Gew.-% Propylenglycol (1,2 USP)
4.00 Gew.-% Glycerin (99%ig)
0.60 Gew.-% Sorbital T 20 P
0.08 Gew.-% Menthol (kristallin)
25.00 Gew.-% Cosmetol (96%ig)
1.50 Gew.-% Deosent/DeoPlex Clear
0.60 Gew.-% Dexpanthenol

### 1.3 Herstellung einer transpirationshemmenden Zusammensetzung als Roll-On mit hoher Wirkdauer

In einen Rührbehälter wurden zunächst 219.125 g temperiertes Wasser (50°C), 1.500 g Natrium-Hexametaphosphat, 55.000 g Weinsäure sowie 12.500 g Triethanolamin (99%ig) hinzugegeben. Danach ließ man den Rührbehälter während eines Zeitraumes von 3 min. rühren. Die erhaltene Lösung wurde anschließend in einem separaten Behälter filtriert und anschließend in den Rührbehälter zurückgegeben.

Anschließend wurden 15.000 g Harnstoff, 2.500 g Allantoin, 45.000 g Diglycerin sowie 3.000 g Sorbital T 20 P hinzugegeben. Man ließ den Rührbehälter während eines Zeitraumes von 6 min. rühren, wobei während des Rührvorganges 0.500 g Tylose H 4000 NG4 hinzugegeben wurden.

Anschließend wurden 0.375 g Menthol (kristallin), 125.000 g Cosmetol (96%ig), 10.000 g Xiameter (PMX-245), 7.500 g Deosent/DeoPlex Clear sowie 3.000 g Dexpanthenol hinzugegeben. Danach ließ man den Rührbehälter abermals während eines Zeitraumes von 6 min. rühren.

Man erhielt eine farblose, klar aussehende, nach Alkohol riechende viskose Zusammensetzung, welche die obengenannten Inhaltsstoffe in den folgenden Anteilen aufwies:
43.82 Gew.-% Wasser
0.30 Gew.-% Natrium-Hexametaphosphat
11.00 Gew.-% Weinsäure
2.50 Gew.-% Triethanolamin (99%ig)
3.00 Gew.-% Harnstoff
0.50 Gew.-% Allantoin
9.00 Gew.-% Diglycerin
0.60 Gew.-% Sorbital T 20 P
0.10 Gew.-% Tylose H 4000 NG4
0.08 Gew.% Menthol (kristallin)
25.00 Gew.% Cosmetol (96%ig)
2.00 Gew.-% Xiameter PMX-245
1.50 Gew.-% Deosent/DeoPlex Clear
0.60 Gew.-% Dexpanthenol.

### 1.4 Herstellung eines transpirationshemmenden Gels mit hoher Wirkdauer

In einem Rührbehälter wurden zunächst 129.625 g temperiertes Wasser (50°C), 1.500 g Natrium-Hexametaphosphat, 5.000 g Esaflor® HDR sowie 12.500 g Triethanolamin (99%ig) hinzugegeben. Dann ließ man den Rührbehälter während eines Zeitraumes von 3 min. rühren. Anschließend wurden 15.000 g Harnstoff, 2.500 g Allantoin, 40.000 g Glycerin (99%ig), 3.000 g Sorbital T 20 P sowie 125.000 g Cosmetol (96%ig) hinzugegeben. Danach ließ man den Rührbehälter während eines Zeitraumes von 6 min. rühren.

In einem separaten Behälter wurden 100.000 g temperiertes Wasser (50°C) und 55.000 g Weinsäure miteinander unter Erhalt einer Lösung gemischt. Die erhaltene Lösung wurde sodann filtriert und anschließend in den Rührbehälter gegeben.

Anschließend wurden 0.375 g Menthol (kristallin), 7.500 g Deosent/DeoPlex Clear sowie 3.000 g Dexpanthenol zu dem Rührbehälter hinzugegeben. Danach wurde der Rührbehälter abermals während eines Zeitraumes von 6 min. gerührt.

Man erhielt eine farblose, minimal trübe, nach Alkohol riechende und hochviskose, gelartige Zusammensetzung, welche die obengenannten Inhaltsstoffe in den folgenden Anteilen aufwies:
45.92 Gew.-% Wasser
0.30 Gew.-% Natrium-Hexametaphosphat
1.00 Gew.-% Esaflor® HDR
2.50 Gew.% Triethanolamin (99%ig)
3.00 Gew.-% Harnstoff
0.50 Gew.% Allantoin
8.00 Gew.-% Glycerin (99%ig)
0.60 Gew.-% Sorbital T 20 P
25.00 Gew.-% Cosmetol (96%ig)
11.00 Gew.-% Weinsäure
0.08 Gew.-% Menthol (kristallin)
1.50 Gew.-% Deosent/DeoPlex Clear
0.60 Gew.-% Dexpanthenol

### 1.5 Herstellung einer weiteren transpirationshemmenden Zusammensetzung zur Verwendung als Pumpspray mit 48h Wirkdauer

In einem Rührbehälter wurden zunächst 194.25 g temperiertes Wasser (50°C), 35.00 g Weinsäure und 12.50 g Triethanolamin (99%ig) hinzugegeben. Danach ließ man den Rührbehälter während eines Zeitraums von 3 Minuten rühren.

Anschließend wurden 2.00 g Allantoin, 1.50 g Harnstoff, 45.00 g Glycerin (99%ig) und 10.00 g Glycerycaprylat hinzugegeben. Man ließ den Rührbehälter für weitere 6 Minuten rühren. Danach wurden 3.75 g Deosent bzw. Deoplex Clear, 7.50 g Dexpanthenol und 188.50 g Cosmetol (96%ig) hinzugegeben. Man ließ den Rührbehälter abermals während eines Zeitraums von 6 Minuten rühren.

Es wurde eine farblose niedrigviskose Lösung erhalten, welche die oben genannten Inhaltsstoffe in den folgenden Anteilen aufwies:
38.85 Gew.-% Wasser
7.00 Gew.-% Weinsäure
2.50 Gew.-% Triethanolamin (99%ig)
0.40 Gew.-% Allantoin
0.30 Gew.-% Harnstoff
9.00 Gew.-% Glycerin (99%ig)
2.00 Gew.-% Glycerycaprylat
0.75 Gew.-% Deosent/Deoplex Clear
1.50 Gew.-% Dexpanthenol
37.70 Gew.-% Cosmetol (96%ig)

### 1.6 Herstellung einer weiteren transpirationshemmenden Roll-On-Zusammensetzung mit 48h Wirkdauer

In einem Rührbehälter wurden zunächst 193.50 g temperiertes Wasser (50°C), 7.50 g Esaflor HDR, 35.00 g Weinsäure sowie 12.50 g Triethanolamin (99%ig) hinzugegeben. Danach ließ man den Rührbehälter während eines Zeitraums von 3 Minuten rühren. Die erhaltene Lösung wurde anschließend in einem separaten Behälter filtriert und danach in den Rührbehälter zurückgegeben.

Anschließend wurden 2.00 g Allantoin, 1.50 g Harnstoff, 45.00 g Glycerin (99%ig) sowie 10.00 g Glycerycaprylat hinzugegeben. Man ließ den Rührbehälter während eines Zeitraums von 6 Minuten rühren. Anschließend wurden 3.75 g Deosent/Deoplex Clear, 7.50 g Dexpanthenol und 188.50 g Cosmetol (96%ig) hinzugegeben. Danach ließ man den Rührbehälter abermals während eines Zeitraums von 6 Minuten rühren.

Man erhielt eine farblose, niedrigviskose Lösung, welche die oben genannten Inhaltsstoffe in den folgenden Anteilen aufwies:
38.70 Gew.-% Wasser
0.15 Gew.-% Esaflor HDR
7.00 Gew.-% Weinsäure
2.50 Gew.-% Triethanolamin (99%ig)
0.40 Gew.-% Allantoin
0.30 Gew.-% Harnstoff
9.00 Gew.-% Glycerin (99%ig)
2.00 Gew.-% Glycerycaprylat
0.75 Gew.-% Deosent/Deoplex Clear
1.50 Gew.-% Dexpanthenol
37.70 Gew.-% Cosmetol (96%ig)

### 1.7 Herstellung einer weiteren transpirationshemmenden, duftstoffhaltigen Zusammensetzung zur Verwendung als Pumpspray mit 24h Wirkdauer

In einem Rührbehälter wurden zunächst 242.50 g temperiertes Wasser (50°C) und 18.75 g Weinsäure hinzugegeben. Danach ließ man den Rührbehälter während eines Zeitraums von 3 Minuten rühren.

Anschließend wurden 2.50 g Allantoin, 1.00 g Harnstoff, 45.00 g Glycerin (99%ig) sowie 6.25 g Glycerycaprylat hinzugegeben. Danach ließ man den Rührbehälter während eines Zeitraumes von 6 Minuten rühren.

Anschließend wurden 8.25 g Dexpanthenol, 175.00 g Cosmetol (96%ig) sowie 0.75 g einer Parfümskomposition hinzugegeben. Man ließ den Rührbehälter abermals während eines Zeitraums von 6 Minuten rühren.

Man erhielt eine farblose, niedrigviskose Lösung, welche die oben genannten Inhaltsstoffe in den folgenden Anteilen aufwies:
48.50 Gew.-% Wasser
3.75 Gew.-% Weinsäure
0.50 Gew.-% Allantoin
0.20 Gew.-% Harnstoff
9.00 Gew.-% Glycerin (99%ig)
1.25 Gew.-% Glycerycaprylat
1.65 Gew.-% Dexpanthenol
35.00 Gew.-% Cosmetol (96%ig)
0.15 Gew.-% Parfümkomposition

### 1.8 Herstellung einer weiteren transpirationshemmenden, duftstoffhaltigen Zusammensetzung zur Verwendung als Roll-On mit 24h Wirkdauer

In einem Rührbehälter wurden zunächst 243.00 g temperiertes Wasser (50°C), 0.75 g Esaflor HDR und 20.00 g Weinsäure hinzugegeben. Danach ließ man den Rührbehälter während eines Zeitraums von 3 Minuten rühren.

Anschließend wurden 2.50 g Allantoin, 1.00 g Harnstoff, 42.50 g Glycerin (99%ig) und 6.25 g Glycerycaprylat hinzugegeben. Danach ließ man den Rührbehälter während eines Zeitraums von 6 Minuten rühren.

Anschließend wurden 8.25 g Dexpanthenol, 175.00 g Cosmetol (96%ig) sowie 0.75 g einer Parfümkomposition hinzugegeben. Man ließ den Rührbehälter abermals während eines Zeitraums von 6 Minuten rühren.

Man erhielt eine farblose, mittelviskose Lösung, welche die oben genannten Inhaltsstoffe in den folgenden Anteilen aufwies:
48.60 Gew.-% Wasser
0.15 Gew.-% Esaflor HDR
4.00 Gew.-% Weinsäure
0.50 Gew.-% Allantoin
0.20 Gew.-% Harnstoff
8.50 Gew.-% Glycerin (99%ig)
1.25 Gew.-% Glycerycaprylat
1.65 Gew.-% Dexpanthenol
35.00 Gew.-% Cosmetol (96%ig)
0.15 Gew.-% Parfümkomposition

### 2. Testen der gemäß Beispielen 1.1 bis 1.8 hergestellten Zusammensetzungen auf ihre transpirationshemmende Wirkung

### 2.1 Person mit starkem Achselschweiß

Die gemäß den Beispielen 1.1 - 1.8 hergestellten Zusammensetzungen wurden jeweils an Personen verschiedenen Geschlechts im Alter von 15 bis 48 Jahren getestet, welche an regelmäßigem starkem Achselschweiß litten. Die Auftragung der Zusammensetzungen auf die betroffenen Achselpartien führte jeweils zu einer signifikanten Verringerung der Schweißmenge sowie zu einem Ausbleiben von Schweißgeruch. Die gewünschte Wirkung hielt im Falle der gemäß den Beispielen 1.1, 1.3 und 1.4 hergestellten Zusammensetzungen über einen Zeitraum von bis zu 48 Stunden an. Im Falle der gemäß den Beispielen 1.2, 1.7 und 1.8 hergestellten Zusammensetzungen hielt die gewünschte Wirkung über einen Zeitraum von bis zu 18 Stunden an. Im Falle der gemäß den Beispielen 1.5 und 1.6 hergestellten Zusammensetzungen hielt die gewünschte Wirkung über einen Zeitraum von über 24 Stunden an.

### 2.2 Person mit normalem Achselschweiß

Die gemäß den Beispielen 1.1 - 1.8 hergestellten Zusammensetzungen wurden jeweils an Personen verschiedenen Geschlechts im Alter von 16 bis 49 Jahren mit normalem Achselschweiß getestet. Die Auftragung der Zusammensetzungen auf die betroffenen Achselpartien führte jeweils zu einer signifikanten Verringerung der Schweißmenge sowie zu einem Ausbleiben von Schweißgeruch. Bei den gemäß Beispielen 1.1, 1.3 und 1.4 hergestellten Zusammensetzungen hielt die gewünschte Wirkung über einen Zeitraum von über 48 Stunden an. Bei den gemäß Beispielen 1.2, 1.7 und 1.8 hergestellten Zusammensetzungen hielt die gewünschte Wirkung über einen Zeitraum von bis zu 24 Stunden an. Bei den gemäß Beispielen 1.5 und 1.6 hergestellten Zusammensetzungen hielt die gewünschte Wirkung über einen Zeitraum von bis zu 48 Stunden an.

## Patentansprüche

1. Aluminiumsalzfreie, kosmetische Antitranspirant-Zusammensetzung, enthaltend wenigstens einen Antitranspirant-Wirkstoff, welcher ausgewählt ist aus der Gruppe bestehend aus Hydroxycarbonsäure, Hydroxycarbonsäuresalz und Mischungen davon, **dadurch gekennzeichnet, dass** der wenigstens eine Antitranspirant-Wirkstoff einen Anteil von 2.5 Gew.-% bis 40 Gew.-%, bezogen auf das Gesamtgewicht der Zusammensetzung, und die Zusammensetzung einen Ethanolanteil von 10 Gew.% bis 50 Gew.-%, bezogen auf das Gesamtgewicht der Zusammensetzung, aufweist, wobei der wengistens eine Antitranspirant-Wirkstoff ausgewählt ist aus der Gruppe bestehend aus Weinsäure, Weinsäursesalz und Mischungen davon.

2. Antitranspirant-Zusammensetzung nach Anspruch 1, **dadurch gekennzeichnet, dass** der wenigstens eine Antitranspirant-Wirkstoff einen Anteil von 5 Gew.-% bis 30 Gew.-%, bevorzugt 5 Gew.-% bis 20 Gew.-%, aufweist, bezogen auf das Gesamtgewicht der Zusammensetzung.

3. Antitranspirant-Zusammensetzung nach Anspruch 1 oder 2, **dadurch gekennzeichnet, dass** die Zusammensetzung ferner wenigstens ein Trägerfluid enthält, welches ausgewählt ist aus der Gruppe bestehend aus Isopropanol, 2-Methyl-propanol-2, Wasser und Mischungen davon.

4. Antitranspirant-Zusammensetzung nach einem der vorhergehenden Ansprüche, **dadurch gekennzeichnet, dass** die Zusammensetzung einen Ethanolanteil von 15 Gew.-% bis 40 Gew.-%, bevorzugt 20 Gew.-% bis 30 Gew.-%, aufweist, bezogen auf das Gesamtgewicht der Zusammensetzung.

5. Antitranspirant-Zusammensetzung nach einem der vorhergehenden Ansprüche, **dadurch gekennzeichnet, dass** die Zusammensetzung einen Wasseranteil von 10 Gew.-% bis 75 Gew.-%, bevorzugt 35 Gew.-% bis 50 Gew.-%, aufweist, bezogen auf das Gesamtgewicht der Zusammensetzung.

6. Antitranspirant-Zusammensetzung nach einem der vorhergehenden Ansprüche, **dadurch gekennzeichnet, dass** die Zusammensetzung ferner ein Saccharomyces Ferment enthält.

7. Antitranspirant-Zusammensetzung nach Anspruch 6 **dadurch gekennzeichnet, dass** das Ferment einen Anteil von 0.5 Gew.-% bis 5 Gew.-%, insbesondere 0.75 Gew.-% bis 4 Gew.-%, bevorzugt 1 Gew.-% bis 3 Gew.-%, aufweist, bezogen auf das Gesamtgewicht der Zusammensetzung.

8. Antitranspirant-Zusammensetzung nach einem der vorhergehenden Ansprüche, **dadurch gekennzeichnet, dass** sie auf 100 Gew.-% folgende Inhaltsstoffe enthält:
- 5 Gew.-% bis 30 Gew.-%, bevorzugt 5 Gew.-% bis 20 Gew.-%, des wenigstens einen Antitranspirant-Wirkstoffes,
- 10 Gew.-% bis 50 Gew.-%, bevorzugt 20 Gew.-% bis 30 Gew.-%, Ethanol,
- 0.5 Gew.-% bis 5 Gew.-%, bevorzugt 1 Gew.-% bis 3 Gew.-%, Saccharomyces Ferment,
- 0 Gew.-% bis 2.5 Gew.-%, bevorzugt 2 Gew.-% bis 2.5 Gew.-%, wenigstens eines pH-Regulators,
- 0 Gew.-% bis 2.5 Gew.-%, bevorzugt 0.2 Gew.-% bis 0.8 Gew.-%, wenigstens eines Komplexbildners,
- 0 Gew.-% bis 2.0 Gew.-%, bevorzugt 0.01 Gew.-% bis 0.5 Gew.-%, wenigstens eines Viskositätsregulators bzw. Filmbildners,
- 0 Gew.-% bis 2 Gew.-%, bevorzugt 0.2 Gew.-% bis 1 Gew.-%, wenigstens eines Tensids,
- 0 Gew.-% bis 30.5 Gew.-%, bevorzugt 5.5 Gew.-% bis 16.5 Gew.-%, wenigstens eines Hautpflegestoffes,
- 0 Gew.-% bis 0.2 Gew.-%, bevorzugt 0.05 Gew.-% bis 0.1 Gew.-%, wenigstens eines Duftstoffes und
- 10 Gew.-% bis 75 Gew.-%, bevorzugt 35 Gew.% bis 50 Gew.%, Wasser.

9. Antitranspirant-Zusammensetzung nach einem der vorhergehenden Ansprüche, **dadurch gekennzeichnet, dass** die Zusammensetzung als Lösung, Suspension, Gel, insbesondere Hydrogel, Creme, Paste, Salbe, Roll-on-Zusammensetzung, Stift, Spray-Sud, Pumpspray oder in Kombination mit einem Treibmittel als Aerosolspray konfektioniert ist.

10. Kosmetisches Produkt, enthaltend oder bestehend aus einer Zusammensetzung nach einem der Ansprüche 1 bis 9.

## Claims

1. Cosmetic antiperspirant composition free of aluminum salt, comprising at least one active antiperspirant ingredient selected from the group consisting of hydroxycarboxylic acid, hydroxycarboxylic salt, and mixtures thereof,
**characterized in that**
the at least one active antiperspirant ingredient has a fraction of 2.5 wt% to 40 wt%, based on the total weight of the composition, and the composition has an ethanol fraction of 10 wt% to 50 wt%, based on the total weight of the composition, wherein the at least one active antiperspirant ingredient is selected from the group consisting of tartaric acid, tartaric salt, and mixtures thereof.

2. Antiperspirant composition according to claim 1, **characterized in that** the at least one active antiperspirant ingredient has a fraction of 5 wt% to 30 wt%, preferably 5 wt% to 20 wt%, based on the total weight of the composition.

3. Antiperspirant composition according to claim 1 or 2, **characterized in that** the composition further comprises at least one carrier fluid selected from the group consisting of isopropanol, 2-methylpropan-2-ol, water, and mixtures thereof.

4. Antiperspirant composition according to any of the preceding claims, **characterized in that** the composition has an ethanol fraction of 15 wt% to 40 wt%, preferably 20 wt% to 30 wt%, based on the total weight of the composition.

5. Antiperspirant composition according to any of the preceding claims, **characterized in that** the composition has a water fraction of 10 wt% to 75 wt%, preferably 35 wt% to 50 wt%, based on the total weight of the composition.

6. Antiperspirant composition according to any of the preceding claims, **characterized in that** the composition further comprises a Saccharomyces ferment.

7. Antiperspirant composition according to claim 6, **characterized in that** the ferment has a fraction of 0.5 wt% to 5 wt%, more particularly 0.75 wt% to 4 wt%, preferably 1 wt% to 3 wt%, based on the total weight of the composition.

8. Antiperspirant composition according to any of the preceding claims, **characterized in that** it contains to 100 wt% the following ingredients:
- 5 wt% to 30 wt%, preferably 5 wt% to 20 wt%, of the at least one active antiperspirant ingredient,
- 10 wt% to 50 wt%, preferably 20 wt% to 30 wt%, of ethanol,
- 0.5 wt% to 5 wt%, preferably 1 wt% to 3 wt%, of Saccharomyces ferment,
- 0 wt% to 2.5 wt%, preferably 2 wt% to 2.5 wt%, of at least one pH regulator,
- 0 wt% to 2.5 wt%, preferably 0.2 wt% to 0.8 wt%, of at least one complexing agent,
- 0 wt% to 2.0 wt%, preferably 0.01 wt% to 0.5 wt%, of at least one viscosity regulator and/or film-former,
- 0 wt% to 2 wt%, preferably 0.2 wt% to 1 wt%, of at least one surfactant,
- 0 wt% to 30.5 wt%, preferably 5.5 wt% to 16.5 wt%, of at least one skincare substance,
- 0 wt% to 0.2 wt%, preferably 0.05 wt% to 0.1 wt%, of at least one fragrance, and
- 10 wt% to 75 wt%, preferably 35 wt% to 50 wt%, of water.

9. Antiperspirant composition according to any of the preceding claims, **characterized in that** the composition is formulated as a solution, suspension, gel, more particularly hydrogel, cream, paste, salve, roll-on composition, stick, spray sud, pump spray or, in combination with a propellant, as an aerosol spray.

10. Cosmetic product comprising or consisting of a composition as claimed in any of claims 1 to 9.

## Revendications

1. Composition cosmétique antitranspirante sans sel d'aluminium, contenant au moins un principe actif antitranspirant, qui est choisi dans le groupe constitué par un acide hydroxycarboxylique, un sel d'acide hydroxycarboxylique et des mélanges correspondants, **caractérisée en ce que** l'au moins un principe actif antitranspirant présente une proportion de 2,5 % en poids à 40 % en poids, par rapport au poids total de la composition, et la composition présente une proportion d'éthanol de 10 % en poids à 50 % en poids, par rapport au poids total de la composition, l'au moins un principe actif antitranspirant étant choisi dans le groupe constitué par l'acide tartrique, un sel d'acide tartrique et des mélanges correspondants.

2. Composition antitranspirante selon la revendication 1, **caractérisée en ce que** l'au moins un principe actif antitranspirant présente une proportion de 5 % en poids à 30 % en poids, préférablement de 5 % en poids à 20 % en poids, par rapport au poids total de la composition.

3. Composition antitranspirante selon la revendication 1 ou 2, **caractérisée en ce que** la composition contient en outre au moins un fluide de support, qui est choisi dans le groupe constitué par l'isopropanol, le 2-méthyl-propanol-2, l'eau et des mélanges correspondants.

4. Composition antitranspirante selon l'une quelconque des revendications précédentes, **caractérisée en ce que** la composition présente une proportion d'éthanol de 15 % en poids à 40 % en poids, préférablement de 20 % en poids à 30 % en poids, par rapport au poids total de la composition.

5. Composition antitranspirante selon l'une quelconque des revendications précédentes, **caractérisée en ce que** la composition présente une proportion d'eau de 10 % en poids à 75 % en poids, préférablement de 35 % en poids à 50 % en poids, par rapport au poids total de la composition.

6. Composition antitranspirante selon l'une quelconque des revendications précédentes, **caractérisée en ce que** la composition comprend en outre un ferment de type Saccharomyces.

7. Composition antitranspirante selon la revendication 6, **caractérisée en ce que** le ferment présente une proportion de 0,5 % en poids à 5 % en poids, en particulier de 0,75 % en poids à 4 % en poids, préférablement de 1 % en poids à 3 % en poids, par rapport au poids total de la composition.

8. Composition antitranspirante selon l'une quelconque des revendications précédentes, **caractérisée en ce qu'**elle contient les ingrédients suivants jusqu'à 100 % en poids :
- 5 % en poids à 30 % en poids, préférablement 5 % en poids à 20 % en poids, de l'au moins un principe actif antitranspirant,
- 10 % en poids à 50 % en poids, préférablement 20 % en poids à 30 % en poids, d'éthanol,
- 0,5 % en poids à 5 % en poids, préférablement 1 % en poids à 3 % en poids, de ferment de type Saccharomyces,
- 0 % en poids à 2,5 % en poids, préférablement 2 % en poids à 2,5 % en poids, d'au moins un régulateur du pH,
- 0 % en poids à 2,5 % en poids, préférablement 0,2 % en poids à 0,8 % en poids, d'au moins un agent complexant,
- 0 % en poids à 2,0 % en poids, préférablement 0,01 % en poids à 0,5 % en poids, d'au moins un régulateur de viscosité ou, selon le cas, d'un agent filmogène,
- 0 % en poids à 2 % en poids, préférablement 0,2 % en poids à 1 % en poids, d'au moins un tensioactif,
- 0 % en poids à 30,5 % en poids, préférablement 5,5 % en poids à 16,5 % en poids, d'au moins un produit de soin de la peau,
- 0 % en poids à 0,2 % en poids, préférablement 0,05 % en poids à 0,1 % en poids, d'au moins un parfum et
- 10 % en poids à 75 % en poids, préférablement 35 % en poids à 50 % en poids, d'eau.

9. Composition antitranspirante selon l'une quelconque des revendications précédentes, **caractérisée en ce que** la composition est confectionnée sous forme de solution, de suspension, de gel, en particulier d'hydrogel, de crème, de pâte, de pommade, de composition d'applicateur à bille, de crayon, de décoction en spray, de vaporisateur ou en combinaison avec un agent propulseur en tant que spray aérosol.

10. Produit cosmétique, contenant ou étant constitué d'une composition selon l'une quelconque des revendications 1 à 9.
